# EUROPEAN PATENT APPLICATION

(11) **EP 0 870 510 A2**
(43) Date of publication of application: **14.10.1998**
(21) Application number: 98302691.5
(22) Date of filing: 06.04.1998
(51) Int. Cl.: A61K 45/06

(54) **Synergistic combination comprising cryptophycin derivatives and microtubule synergizing agents**

(30) Priority: 11.04.1997 US 43581 P
(71) Applicant: ELI LILLY AND COMPANY, Indianapolis, Indiana 46285 (US)
(72) Inventor: Shih, Chuan, Carmel, Indiana 46033 (US); Williams, Daniel Charles, Fishers, Indiana 46038 (US)
(74) Representative: Denholm, Anna Marie

(57) **Abstract**

A composition for providing a synergistic treatment for cancer comprising a compound selected from the group consisting of Compound I, Compound II, Compound III, Compound IV and Compound V and one or more microtubule synergizing agents is disclosed. Also, a method of treating cancer using a therapeutically effective amount of a compound selected from the group consisting of Compound I, Compound II, Compound III, Compound IV and Compound V and one or more microtubule synergizing agents or radiation therapy is also disclosed.

## Description

### BACKGROUND OF THE INVENTION

This invention relates to the fields of pharmaceutical and organic chemistry and provides a composition that can provide a synergistic effect for the treatment of cancer or hyperproliferative growth in a mammal comprising cryptophycin compounds and a microtubule synergizing agent. An additional useful composition which can provide a synergistic therapy for the treatment of cancer is cryptophycin compounds used in combination with radiation therapy.

Certain cryptophycin compounds are now known to have antiproliferative activity and are believed to be useful for the treatment of cancer, neoplasms and hyperproliferative cell growth. Applicants have discovered a composition which can provide an unexpectedly synergistic effect for the treatment for cancer, neoplasm, and hyperproliferative cell growth. Such composition can provide more treatment options for the physician to choose from and can provide an effect which genuinely synergistic.

There is a great need for additional treatment options and the present invention can address this long felt need for greater options to provide the oncology patient with a more effective anti-tumor treatment.

### SUMMARY OF THE INVENTION

The presently claimed invention provides a composition useful for the treatment of a mammal afflicted with a condition selected from the group consisting of cancer, neoplasm, and hyperproliferative cell growth, comprising a compound selected from the group consisting of Compound I, Compound II, Compound III, Compound IV and Compound V. 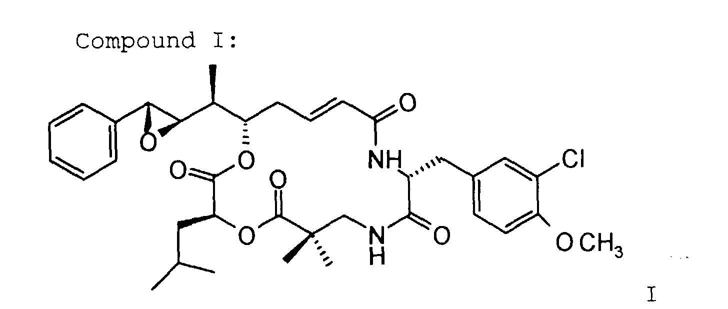 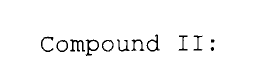 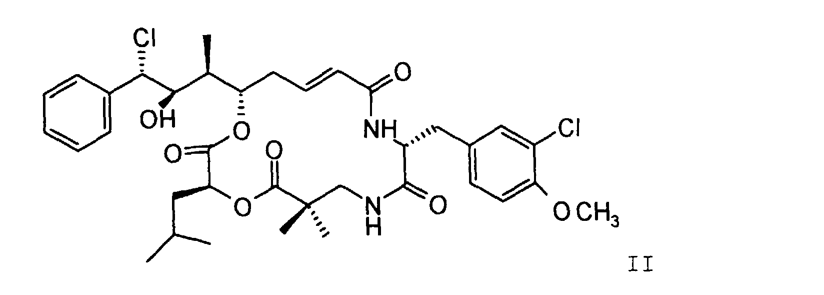 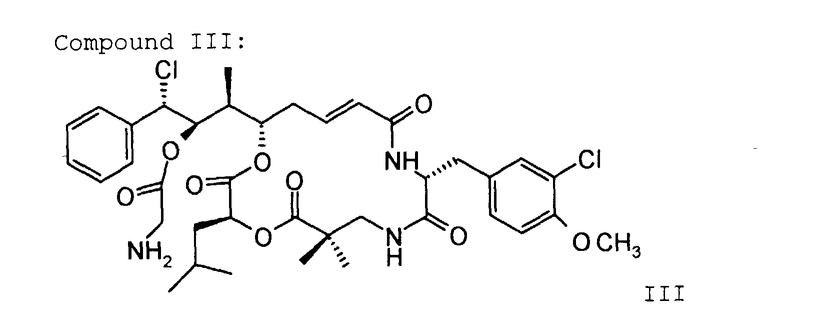 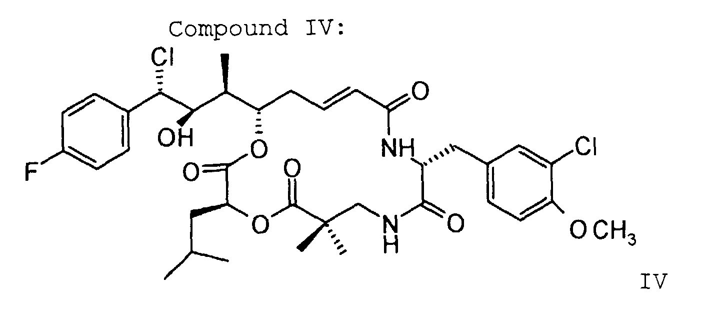 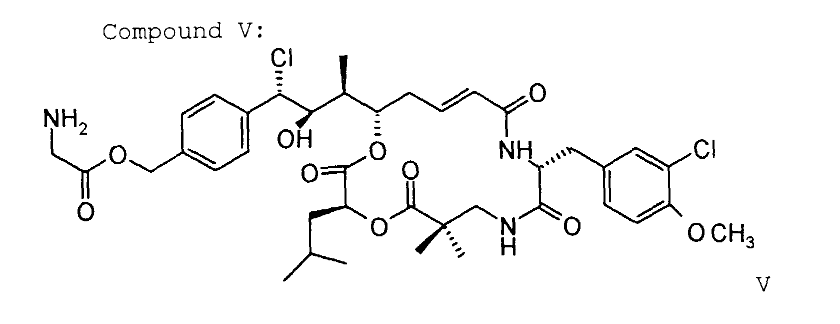 or a pharmaceutically acceptable salt thereof; and one or more a microtubule synergizing agents.

The present invention provides a method for treating a mammal afflicted with cancer comprising administering a therapeutically effective amount of a compound selected from the group consisting of 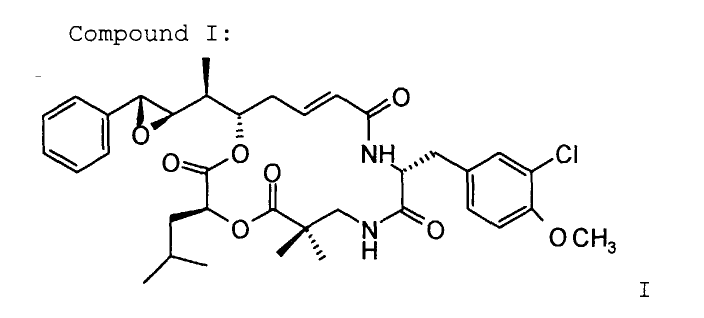 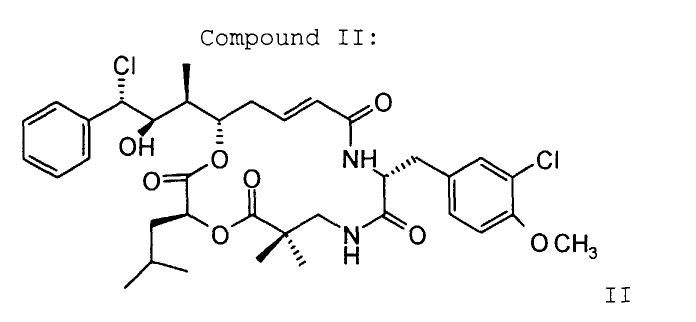 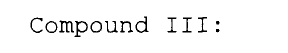 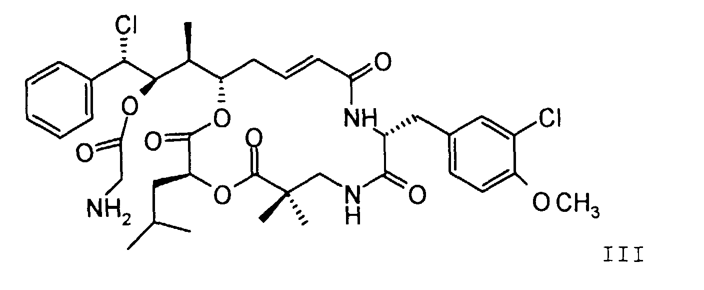 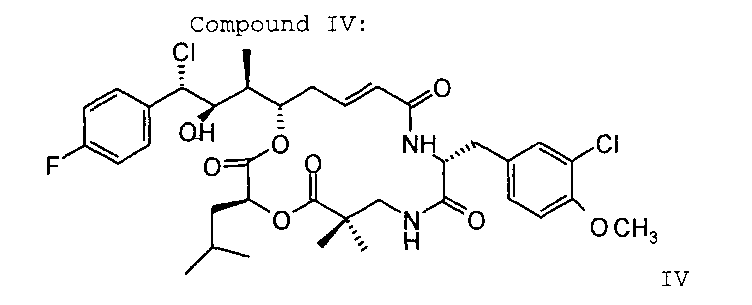 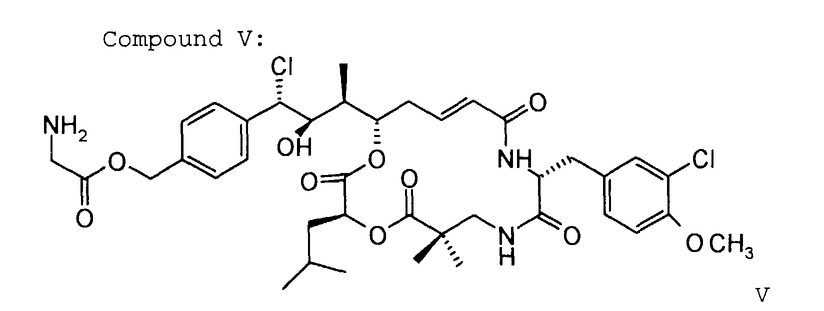 or a pharmaceutically acceptable salt thereof; and one or more a microtubule synergizing agents.

The present invention further provides a method for treating a mammal with cancer comprising administering a therapeutically effective amount of a compound selected from 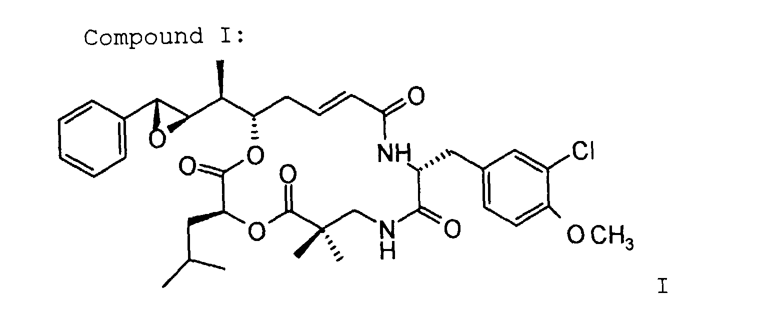 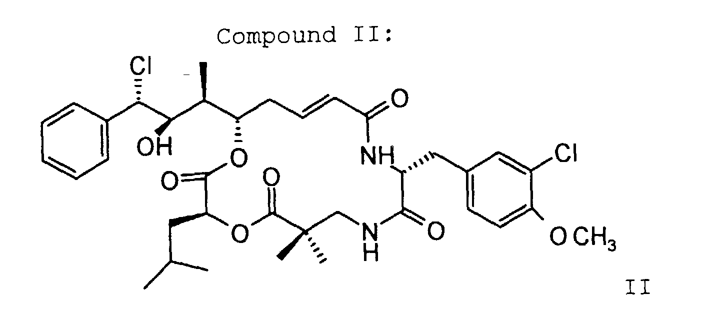 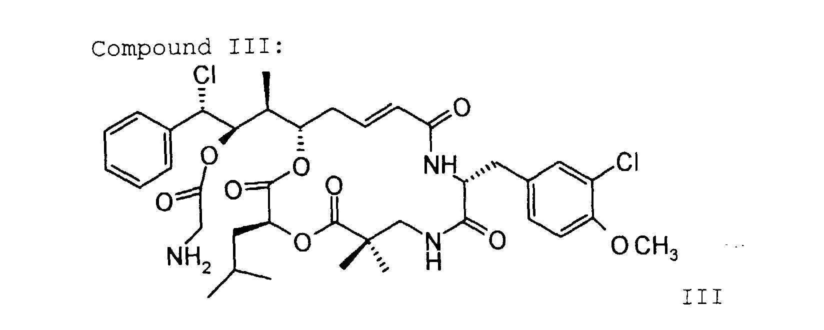 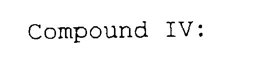 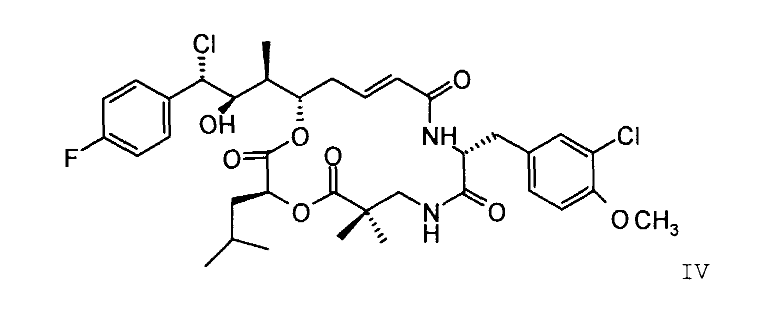 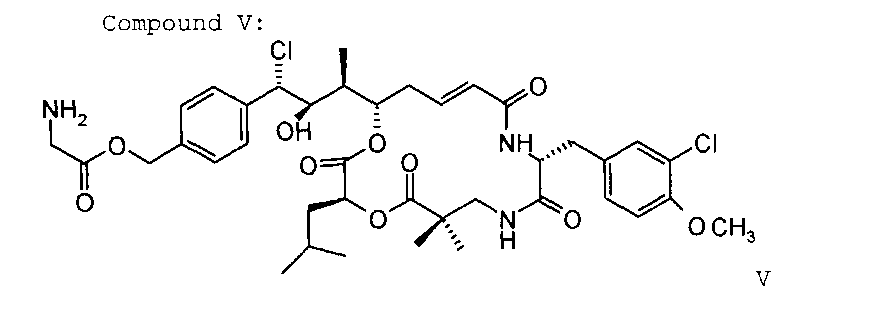 or a pharmaceutically acceptable salt thereof; prior to or at the time of radiation therapy.

Further, the present invention includes a composition for the treatment of a mammal afflicted with cancer comprising a related cryptophycin compound and one or more microtubule synergizing agents.

### DETAILED DESCRIPTION OF THE INVENTION

As used herein, the term "a microtubule synergizing agent" refers to an agent that can act on tumor cells at a microtubule site that is different than the microtubule site acted on by Compounds I-V or related cryptophycin compounds. Examples of "microtubule synergizing agents" include estramustine, paclitaxel, docetaxel (taxotere), the vinca alkaloids, such vinblastine, vincristine, and vinorelbine which are known and available to the skilled artisan.

As used herein the term "radiation therapy" refers to the widely accepted use of radiation to treat cancer. The techniques for administration of the radiation therapy should be consistent with the practices in the art; however, the radiation therapy is administered concurrently with or after the patient in need of such treatment has received a dosage of Compounds I-V, or related cryptophycin compound. The related cryptophycin compound or Compounds I-V can be administered up to twenty four hours prior to radiation; however, such treatment may be made immediately prior to the radiation therapy.

As used herein, the term "mammal" shall refer to the Mammalia class of higher vertebrates. The term "mammal" includes, but is not limited to, mice, dogs, rats, hamsters, guinea pigs, cows, apes and humans. The term "treating" as used herein includes prophylaxis of the named condition or amelioration or elimination of the condition once it has been established. The cryptophycin compounds claimed herein can be useful for vetrinary health purposes as well as for the treatment of a human patient.

The mechanism of action of Compounds I-V and related cryptophycin compounds has been discovered to involve interaction with the microtubules. Surprisingly, Applicants have discovered that the activity of cryptophycin compounds involves antimitotic activity and accumulation of cells in the mitotic phase of the cell cycle. Applicants have discovered that the claimed compositions can synchronize the cancer cells at a cell cycle stage wherein the cancer cells are particularly sensitive to the cryptophycin mechanism of action, providing a synergistic treatment effect that is greater than the expected sum of the two agents would have been. The claimed compositions provide the desired synchronization and the surprising synergistic effect.

As used herein the term "related cryptophycin compounds" refers to a compound of the formula IA 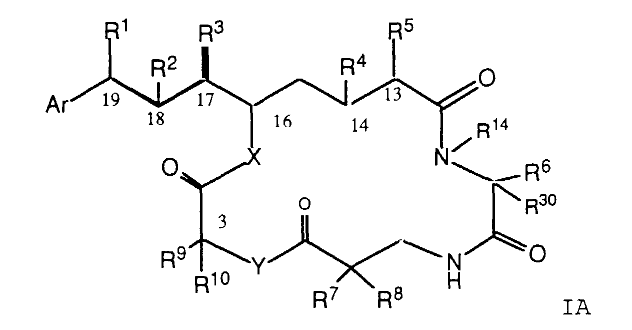 wherein
Ar is phenyl or any simple unsubstituted or substituted aromatic or heteroaromatic group;
R¹ is halogen, SH, amino, monoalkylamino, dialkylamino, trialkylammonium, alkylthio, dialkylsulfonium, sulfate, or phosphate;
R² is OH or SH; or
R¹ and R² may be taken together to form an epoxide ring, and aziridine ring, an episulfide ring, a sulfate ring, or monoalkylphosphate ring; or
R¹ and R² may be taken together to form a second bond between C₁₈ and C₁₉;
R³ is a lower alkyl group;
R⁴ is H;
R⁵ is H;
R⁴ and R⁵ may be taken together to form a second bond between C₁₃ and C₁₄;
R⁶ is benzyl, hydroxybenzyl, alkoxybenzyl, halohydroxybenzyl, dihalohydroxybenzyl, haloalkoxybenzyl, or dihaloalkoxybenzyl group, a substituent selected from the group consisting of B-ring heteroaromatic, substituted heteroaromatic, B-ring (C₁-C₆)alkyl, (C₃-C₈)cycloalkyl, substituted C₃-C₈ cycloalkyl, substituted (C₁-C₆)alkyl, a group of the formula **III'**: 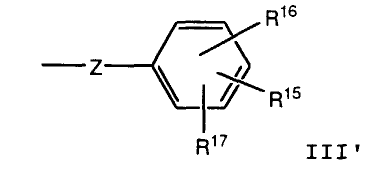 and a group of the formula **III"**: 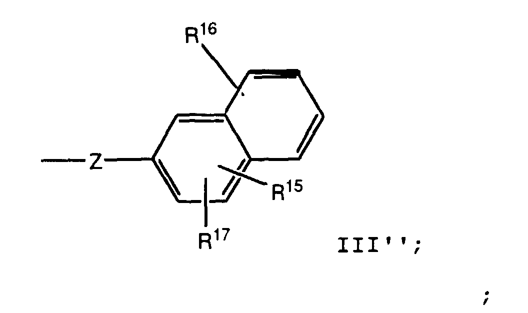
R⁷ is H or a lower alkyl group;
R⁸ is H or a lower alkyl group;
R⁹ is H or a lower alkyl group;
R¹⁰ is H or a lower alkyl group;
R¹⁴ is H or a lower alkyl group;
R¹⁵, R¹⁶, and R¹⁷ are each independently selected from the group consisting of hydrogen, OR¹⁸, halo, NH₂, NO₂, OPO₄H₂, OR¹⁹phenyl, and ZZ;
R¹⁸ is C₁-C₆ alkyl;
R¹⁹ is C₁-C₆ alkyl;
R³⁰ is C₁-C₆ alkyl;
n is 0, 1, or 2;
p is 0, 1, or 2;
m is 0, 1, or 2;
X is O, NH or alkylamino;
Y is C, O, NH, S, SO, SO₂ or alkylamino;
Z is selected from the group consisting of -(CH₂)ₙ-, -(CH₂)p-O-(CH₂)ₘ- and (C₃-C₅)cycloalkyl;
ZZ is selected from the group consisting of an aromatic group and a substituted aromatic group; or
a pharmaceutically acceptable salt or solvate thereof. The compounds of formula IA are disclosed in PCT International Application Publication Number WO 96/40184, published December 19, 1996. The term "related cryptophycin compound" shall also refer to known cryptophycin compounds such as Cryptophycin 1, 3, 5, 13, 15. Such cryptophycin compounds are disclosed in U.S. patent numbers 4,946,835, 4,845,085, 4,845,086, and 4,868,208, which are hereby incorporated by reference. The term shall also refer to other known cryptophycin compounds such as cryptophycin 8.

The most preferred cryptophycin compounds are Compounds I-V.

As used herein, the term "therapeutically effective amount" refers to an amount of (1) a compound selected from Compounds I-V and/or one or more microtubule synergizing agent, (2) a related cryptophycin compound and/or one or more microtubule synergizing agent, (3) a compound selected from Compounds I-V and/or radiation therapy, or (4) a related cryptophycin compound and/or radiation therapy, which is effective, upon single or multiple dose administration to the mammal, in controlling the growth of the neoplasm or in prolonging the survivability of the mammal beyond that expected in the absence of such treatment. As used herein, "controlling the growth" of the neoplasm refers to slowing, interrupting, arresting or stopping its growth and metastases and does not necessarily indicate a total elimination of the neoplasm.

A therapeutically effective amount can be readily determined by the attending diagnostician, as one skilled in the art, by the use of known techniques and by observing results obtained under analogous circumstances. In determining the therapeutically effective amount or dose, a number of factors are considered by the attending diagnostician, including, but not limited to: the species of mammal; its size, age, and general health; the specific disease involved; the degree of or involvement or the severity of the disease; the response of the individual mammal; the particular compound administered; the bioavailability characteristics of the preparation administered; the dose regimen selected; the use of concomitant medication; and other relevant circumstances.

A therapeutically effective amount of radiation therapy can be readily determined by one of ordinary skill in the art. A therapeutically effective amount of a compound selected from Compounds I-V, a related cryptophycin compound and/or microtubule synergizing agent is expected to vary from about 0.001 milligram per kilogram of body weight per day (mg/kg/day) to about 1000 mg/kg/day. Preferred amounts are expected to vary from about 0.01 to about 10 mg/kg/day.

A compound of Compounds I-V, a related cryptophycin compound or a microtubule synergizing agent can be administered to the mammal in any form or mode which makes the compound bioavailable in effective amounts, including oral and parenteral routes. For example, a compound of Compounds I-V, a related cryptophycin compound or a microtubule synergizing agent can be administered orally, subcutaneously, intramuscularly, intravenously, transdermally, intranasally, rectally, and the like. Oral, intravenous or intramuscular administration is generally preferred. One skilled in the art of preparing formulations can readily select the proper form and mode of administration depending upon the particular characteristics of the compound selected, the disease state to be treated, the stage of the disease, and other relevant cicumstances.

The compounds can be administered alone or in the form of a pharmaceutical composition in combination with pharmaceutically acceptable carriers or excipients, the proportion and nature of which are determined by the solubility and chemical properties of the compound selected, the chosen route of administration, and standard pharmaceutical practice. The compounds of the invention, while effective themselves, may be formulated and administered, where appropriate, in the form of their pharmaceutically acceptable acid addition salts for purposes of stability, convenience of crystallization, increased solubility and the like. It is preferred that a composition or formulation of this invention will be used to treat a neoplasm.

In a further preferred embodiment the neoplasms are selected from the group consisting of mammary, small-cell lung, non-small-cell lung, colorectal, leukemia, lymphoma, melanoma, pancreatic adenocarcinoma, central nervous system carcinoma, ovarian, prostate, stomach, myeloma, bladder, renal, neuroendocrine which includes thyroid and non-Hodgkin's disease and Hodgkin's disease neoplasms.

These compositions or formulations can be administered to mammals for veterinary use. For example, domestic animals can be treated in much the same way as a human clinical patient.

In general, the dosage required for therapeutic effect will vary according to the type of use, mode of administration, as well as the particularized requirements of the individual hosts. Typically, dosages will range from about 0.001 to 1000 mg/kg, and more usually 0.01 to 10 mg/kg of the host body weight of the cryptophycin compound; however, a smaller dosage will likely be required for the compositions of this invention. Or, alternatively, dosages within these ranges can be administered as a bolus or I.V. injection, until the desired therapeutic benefits are obtained. Indeed, drug dosage, as well as route of administration, must be selected on the basis of relative effectiveness, relative toxicity, growth characteristics of tumor and effect of the synergistic composition on cell cycle, drug pharmacokinetics, age, sex, physical condition of the patient and prior treatment.

The compositions of this invention, with or without an additional microtubule synergizing agent, may be formulated into therapeutic compositions as natural or salt forms. Pharmaceutically acceptable non-toxic salts include base addition salts which may be derived from inorganic bases such as for example, sodium, potassium, ammonium, calcium, or ferric hydroxides, and such organic bases as isopropylamine, trimethylamine, 2-ethylamino ethanol, histidine, procaine, and the like. Such salts may also be formed as acid addition salts with any free cationic groups and will generally be formed with inorganic acids such as for example, hydrochloric or phosphoric acids or organic acids such as acetic, oxalic, tartaric, mandelic, and the like. Additional excipients which further the invention are provided to the skilled artisan for example in the U.S. *Pharmacopeia.*

The suitability of particular carriers for inclusion in a given therapeutic composition depends on the preferred route of administration. For example, antineoplastic compositions may be formulated for oral administration. Such compositions are typically prepared as liquid solution or suspensions or in solid forms. Oral formulation usually include such additives as binders, fillers, carriers, preservatives, stabilizing agents, emulsifiers, buffers, mannitol, lactose, starch, magnesium stearate, sodium saccharin, cellulose, magnesium carbonate, and the like. These compositions may take the form of solutions, suspensions, tablets, pills, capsules, sustained relsease formulations, or powders, and typically contain 1% to 95% of active ingredient. More preferably, the composition contains from about 2% to about 70% by weight of each active ingredient.

An especially preferred route of administration is injection or infusion.

A formulation according to the invention may be in unit dosage form, for example, unit dosage form for parenteral administration, which will primarily include administration by injection or infusion, especially intramuscularly and intravenous administration. The composition may comprise any of Compounds I-V or related cryptophycin and the synergizing agent in separate dosage forms for adminstration together or sequentially. For example, these constituents and suitable excipients may be presented in separate vials for making up into a solution or suspension for injection or infusion.

The microtubule synergizing agent will generally be administered in an amount sufficient to provide an effect on the activity at the microtubule of the cancer cells.

Compositions of the present invention may be prepared as injectables, either as liquid solutions, suspensions, or emulsions; solid forms suitable for solution in or suspension in liquid prior to injection. Such injectables may be administered subcutaneously, intravenously, intraperitoneally, intramuscularly, intrathecally, or intrapleurally. The active ingredients are often mixed with diluents, carriers, or excipients which are physiologically tolerable and compatible with the active ingredients. Suitable diluents and excipients are for example, water, saline, dextrose, glycerol, or the like and combinations thereof. In addition, if desired, the compositions may contain minor amounts of auxilary substances such as wetting or emulsifying agents, stabilizing or pH buffering agents.

The effectiveness of the claimed compositions can be assessed using standard methods known to the skilled artisan. Examples of such methods are as follows:

The compositions of this invention can be tested in vivo against a broad spectrum of murine and human tumors implanted in mice, including drug resistant tumors. The activity is measured to determine the tumor burden T/C (mean tumor burden in treated animals/mean tumor burden untreated animals). It is most desired that the values are less than 10%. T/C values less than 42% are considered to be active by NCI standards; T/C values that are less than 10% are considered to have excellent activity and potential clinical activity by NCI standards. The gross log kill values are measured as well. Gross log kill is defined as T-C/3.2 Td where T is the median time in days for the tumors of the treated group to reach 750 mg, C is the median time in days for the tumors of the control group to reach 750 mg, and Td is the tumor volume doubling time. Gross log kill values of greater than 2.8, 2.0-2.8, 1.3-1.9, 0.5-0.8, and less than 0.5 with duration of composition treatment of five to twenty days are scored "++++", "+++", "++", "+" and "-" (inactive) respectively. An activity rating of +++ to ++++, which strongly suggests clinical activity, is needed to effect partial or complete regression of 100-300 mg size masses of most transplanted solid tumors of mice.

The interaction of the dosages on log kill can be demonstrated by the Loewe isobologram (S. Loewe, Pharm. Rev. 9, 237-242 (1957).

The solid line connecting the Gross log kill of the cryptophycin compound (alone) and a microtubule synergizing agent or radiation therapy respectively as claimed herein (alone) represents the "Gross log kill addition line" which indicates the expected location of the Gross log kill's for the cryptophycin compound and a microtubule synergizing agent or radiation therapy combinations if simple additivity were to describe their combined effects. The 95% confidence range for the Gross log kill addition line is shown by the area between the broken lines above and below the Gross log kill addition line. Such theory can be applied to the ED50 dosages when the toxicology of the compound permits such measure.

According to Loewe's isobolic theory, if the effects are simply additive to one another, then the expected location of the Gross log kill of Cryptophycin compound and synergizing agent or radiation thereapy component respectively of each fixed dosage ratio would be contained within or overlap the region of the Gross log kill addition line. Combination Gross log kill's located significantly below the Gross log kill addition line would represent unexpectedly enhanced antitumor activity and combination Gross log kill's located above the line would represent unexpected diminished antitumor effect.

One method to establish the significance of such unexpected enhanced or diminished activity is to calculate the best fitting polynomial regression line to the observed Gross log kill's using standard mathematical techniques.

Such experiments demonstrate that compositions comprised of a related cryptophycin compound or Compounds I-V and one or more Synergistic Agents provides a statistically significant synergistic antitumor effect.

Further, such experiments can demonstrate that compositions comprised of a related cryptophycin or Compounds I-V and one or more activating agents provides statistically significant synergistic antitumor effect.

Compounds used in the compositions of this invention can be prepared using the following Examples as a guide. Methods for preparing Compounds I-V are known to the skilled artisan.

### Example 1

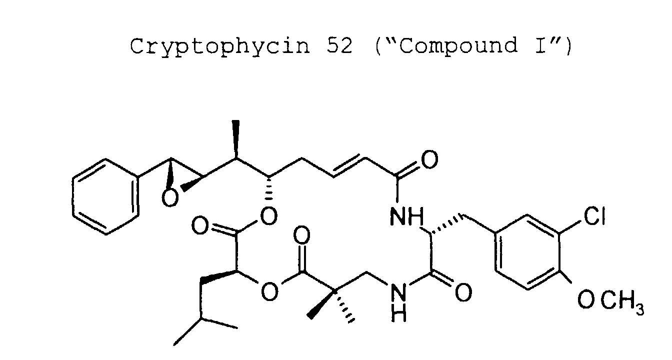

A racemic trans-3-penten-2-ol (933 mg), trifluoroethyl laurate (4.14 g) and porcine pancreatic lipase (PPL, 2.0g) in about 25 ml of anhydrous diethyl ether was stirred for 80 hours. The PPL was then filtered off and washed with ether. The ether filtrate was evaporated and the sticky oil was then subjected to short-path vacuum distillation. The S-trans-3-penten-2-ol (A) was condensed in a liquid nitrogen cooled trap (383mg) and identified using NMR.

The material was then vigorously stirred with tetrabutylammonium hydrogen sulfate and 40% NaOH in water at 0C. Propargyl chloride was added dropwise to the mixture (767 mg). Vigorous stirring was continued overnight after which time the mixture was neutralized with HCL at 0C and the propargyl ether extracted into pentane. The extract was evaporated and the propargyl ether was purified on a short silica column to give propargyl ether B which was characterized using NMR.

An aliquot of butyl lithium hexane solution (2.5 M, 5.1 ml) was evaporated in vacuo and the residue cooled to -90C. A solution of propargyl ether B (454 mg) in 10 ml of tetrahydrofuran was slowly added. After allowing the temperature to increase to room temperature overnight, the reaction mixture was quenched with NH₄Cl solution. Extraction with ether evaporation and purification of the residue gave 322 mg of alcohol C which was characterized by NMR.

To a stirred solution of alcohol C (248 mg) and imidazole (340 mg) in 3 mL of dry DMF was added tert-butyldimethylsilyl chloride (452 mg). After stirring the mixture overnight, 10 mL of NaOH was added to destroy the excess ter-butyldimethylsilyl chloride. The product was extracted into ether and the extract washed, dried and evaporated. Purification of the residue by chromatography on silica gel with hexane gave 457 mg of (3R,4R)-3-tert-butyldimethylsilyloxy-4-methylhept-5-(E)-en-1-yne (D) (96% yield) and was characterized by NMR.

2-methylbutene (1.15 mL 2M folution in THF) was added to 1.1 mL of BH₃ THF solution 1M at -25C and the mixture was stirred in an ice bath for two hours. The temperature was then cooled to -50C and a solution of the TBS derivative (238 mg) in 1 ml of THF was added all at once. The cooling bath was removed and the reaction mixture was allowed to warm to and to remain at room temperature for 1 hour. Then 2.2 M. KH₂PO₄/K₂HPO₄ solution (4.8 mL) and 30% H₂O₂ (0.8 mL) were added at 0C. One hour later, the THF was evaporated and the residue extracted into ether. The dried ether extract was evaporated and the residue chromatographed on silica gel to give 194 mg of aldehyde D, which was characterized by NMR.

To a stirred solution of aldehyde D (0.74 g) and trimethyl phosphonoacetate (632 mg) in 5 mL of THF cooled to -78C was added tetramethylguanidine (435 uL). After 30 minutes the cooling bath was removed and the mixture was stirred for another four hours. The mixture was neutralized with 1 N HCl and the product was extracted into ether. Evaporation of the dried ether extract left a residue which was chromatographed on silica gel to give 0.814 g of E (90% yield) and was characterized by NMR.

Ozone was passed through a solution of methyl ester E (328 mg) and 97 uL of pyridine in 15 mL of CH₂Cl₂ at -78C and the progress of the ozonolysis was monitored by TLC analysis. After the methyl ester had been consumed, about 500mg of zinc dust and 1 mL of glacial acetic acid were added. The temperature was slowly increased to 25 C. The mixture was filtered and the filtrate was washed successively with saturated CuSO₄ and NaHCO3 solutions. After evaporation of the solvent, the crude aldehyde F (249 mg) in the next step without purification.

To a stirred solution of aldehyde F (25.0 mg), in 1.5 mL of THF at -78C was added 0.80 mL of a cold (-78C) mixture of benzyltriphenylphosphonium chloride (268 mg) and n-butyl lithium (280 uL in hexane). After 15 minutes, the cold bath was removed and the stirring was continued for 2 hours. The reaction was quenched with saturated ammonium chloride solution and the THF was evaporated. The concentrate was extracted with hexane and the combined extract was washed with brine, dried, and evaporated. The residual oil was dissolved in 1.5 mL of benzene containing thiophenol (0.02 M) and 1,1'azobis(cyclohexanecarbonitrile) and the mixture was refluxed for 5 hours. After cooling to room temperature, hexane (15 mL) was added and the organic solution was washed successively with 10% NaOH and brine, dried and evaporated. Chromatography of the residue on silica gel let to 24 mg of G.

To a solution of ester G (159 mg) in 7 mL of acetone was added 5 mL of 1N LiOH. The mixture was stirred at 25 C for 3 hours, diluted and acidified to about pH 4 with 1N HCl. The organic layer was separated and washed, dried, and evaporated. Chromatography of the residual oil on silica gel with 40% EtOAc in hexane containing 0.5% AcOH resulted in pure acid H as a pale yellow mobile oil (145 mg).

A sample of the D-chlorotyrosine BOC derivative (160mg) was dissolved in 3 mL neat trifluoroacetic acid and allowed to stand at room temperature for one hour. Removal of the excess reagent under reduced pressure returned the desired amine I as the trifluoroacetate salt (I).

To a stirred solution of acid H (25 mg) in 3 mL of anhydrous DMF under argon was added successively pentafluorodiphenylphosphinate (FDPP, 32 mg), trifluoroacetate salt I (35 mg) and diisopropylethylamine (DIEA, 27mg). Stirring was continued at 25 C for 1 hour and the reaction mixture was extracted. The ether extract was washed, dried, and evaporated. The residual pale yellow oil was subjected to chromatography on silica gel (15% in hexane) to give J as a colorless oil (32 mg). J was characterized by NMR.

To a solution of J (50mg) in 4 mL MeCN was added 400 mL of 49% aq HF and the mixture stirred for 1 hour at 25 C. Extraction followed by washing the ether extract, drying and evaporation gave alcohol K as a colorless foam (40mg).

To a solution of 3-amino-2,2-dimethylpropan-1-ol (L) (3.0g) in 51 mL of a 10% solution of triethylamine in MeOH was added di-tert-butyl dicarbonate (6.7 g) and the mixture was stirred for 1 hour at 25 C. After removal of the solvent, the residue was dissolved in CH₂Cl₂ and the solution was washed and dried. Removal of solvent in vacuo afforded 5.8 g of 3-(tert-butoxycarbonyl)amino-2,2-dimethylpropan-1-ol as a white solid which was directly used for the next step without further purification.

To a solution of alcohol 3-(tert-butoxycarbonyl)amino-2,2-dimethylpropan-1-ol (5.3 g) and sodium periodate (16.6 g) in carbon tetrachloride, acetonitrile and water was added ruthenium trichloride hydrate (122 mg) and the mixture was stirred at 25 C for 1 hour. The mixture was filtered through celite and a saturated solution of potassium carbonate in water was added. The water layer was separated, washed with ether, acidified to pH 2 at 0C and extracted. The combined extracts were washed with saturated NaCl solution and dried. Removal of solvent in vacuo yielded a residue that was first subjected to flash reverse phase chromatography on a C18 silica and then crystallized from ether to give 3.7 g of M as a white solid. M was characterized by NMR.

To a solution of 2.66 gram of L-leucic acid (20 mmol) and 1.74 gram of sodium bicarbonate (20 mmol) in 30 mL water at 0C was added a soltion of 6.44 g tetrabutylammonium chloride (20 mmol) and 1.74 mL of allyl bromide (20mmol). After vigorously stirring the mixture for 24 hours the solvent was evaporated. The residue was passed through a short silica column to give 3.21 g of allyl ester N as a colorless oil.

To a solution of 0.8 g of M (3.7 mmol), 0.76 g of N (4.4 mmol) and 92 mg DMAP in 10 mL of dry CH₂Cl₂ at 0C was added 0.84 g of DCC (4.1 mmol) in CH₂Cl₂. The mixture was stirred at 25 C for 3 hours and filtered. The filtrate was washed with saturated aqueous sodium bicarbonate, dried and evaporated in vacuo. Flash chromatography afforded 1.0 g of pure O as a colorless oil.

To a 10 mL solution of 180 mg of O and 60 mg of tetrakis (triphenylphosphine)palladium in dry THF was slowly added 470 mL of dry morpholine over 10 minutes. After stirring for 50 minutes, 40 mL of ether was added and the solution was washed with 1N HCl. Then the mixture was extracted and the extract acidified and extracted with ether. The ether extract was washed, dried, and evaporated to give P as a colorless mobile oil.

To a solution of alcohol K (80mg), acid P (68mg) and DMAP (4mg) in dry CH2C12 (4 mL) stirred at 0C under an argon atmosphere was added DCC (44 mg) in dry CH₂Cl₂. The mixture was stirred at 0C for 30 minutes, during which time a white precipitate developed, and then allowed to warm to room temperature and stirred for a further 4 hours. The precipitate was filtered off and the filtrate diluted with Et2O and washed. The ethereal layer was dried and evaporated in vacuo to give a waxy solid. Chromatography let to pure Q as a colorless, viscous oil (103 mg).

To the amino acid Q (100mg) was added activated Zinc dust (400mg) and AcOH (4mL). The heterogenous mixture was subjected to sonication for 45 minutes, stirred for a further 90 minutes at room temperature, and then poured onto a pad of Celite. The organic material was washed from the Celite pad with CH₂Cl₂. The solvent was removed in vacuo, leaving the carboxylic acid as a colorless amorphous solid.

Without purification the crude acid was dissolved in trifluoroacetic acid and allowed to sit at room temperature for one hour. After this time excess TFA was removed in vacuo and the resulting amorphous solid was then subjected to chromatographic purification yielding the trifluoroacetate ammonium salt of the desired compound. Repeated lyophilization of an aqueous solution of the salt resulted in the free amino acid R as a colorless amorphous solid.

To a stirred solution of the corresponding amino acid in anhydrous DMF at room temperature was added diisopropylethylamine followed by pentafluorodiphenylphosphinate (FDPP) in DMF (2 mL). The mixture was stirred for 12 hours, EtO was added, and the ether layer washed, dried, and evaporated. The residual waxy solid was further purified by reverse phase chromatography to give Cryptophycin 51 as a colorless amorphous solid.

To a stirred solution of Cryptophycin 51 (75 mg) in anhydrous dichloromethane (7.5 mL) at 0C under argon was added a solution of m-chloroperbenzoic acid (50mg) in dichloromethane (1mL). After 30 minutes the reaction mixture was allowed to warm to room temperature and stirred for a further 12 hours. The solvent was then removed under reduced pressure to give a 1.8:1 mixture of cryptophycins 52 and 53 (by NMR) as an amorphous solid. The mixture of regioisomeric epoxides was dissolved in minimal acetonitrile and subjected to reverse phase chromatography to separate Cryptophycin 52 (37 mg) and Cryptophycin 53. The product was characterized by NMR.

### Example 2

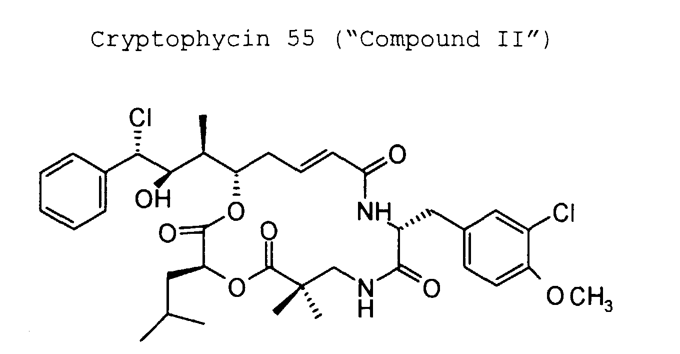

To a solution of Cryptophycin 52 (6mg) in 0.6 mL of 2:1 1,2-dimethoxyethane/water was added 2 uL of 12 N HCl. The solution was allowed to stir at room temperature for 20 hours, neutralized with potassium carbonate, filtered through a 5 m filter, and evaporated. The acetonitrile-soluble material was purified by reversed-phase HPLC on C18 using 4:1 MeOH/water to obtain 3.0 mg of Cryptophycin 55 (48%). The product was characterized using NMR.

### Example 3

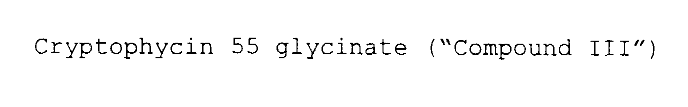

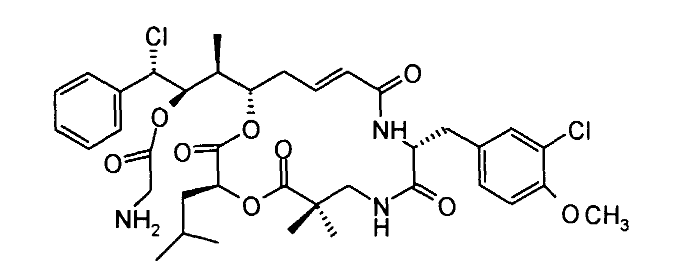

### (a) Preparation of Cryptophycin 55 N-t-Boc-glycinate

To a solution of Cryptophycin 55 (118 mg, 0.167 mmol), N-t-Boc-glycine (44 mg, 0.251 mmol), and 4-dimethylamino pyridine (2.0 mg, 0.0167 mmol) in 490 ml of anhydrous methylene chloride at room temperature was added a solution of 1,3-dicyclohexylcarbodiimide (52 mg, 0.251 mmol) in 67 ml of methylene chloride. After stirring for 50 min, the cloudy white reaction mixture was diluted with ethyl acetate-hexanes (3:1, 1 ml), stirred for 10 min, and filtered through a plug of celite, washing with ethyl acetate-hexanes (3:1). The filtrate and washings were concentrated *in vacuo* to a colorless oil. Chromatography (19 g of flash silica gel, 3:1/ethyl acetate-hexanes) afforded 138 mg (96%) of the title compound as a white foam: 500 MHz ¹H NMR (CDCl₃) d 7.34 (s, 5H), 7.24 (d, 1H, J = 2.0 Hz), 7.23-7.19 (m, 1H), 7.10 (dd, 1H, J = 8.4, 2.0 Hz), 6.88 (d, 1H, J = 8.4 Hz), 6.79-6.70 (m, 1H), 5.77 (d, 1H, J = 13 Hz), 5.50 (d, 1H, J = 8.0 Hz), 5.47 (d, 1H, J = 9.8 Hz), 4.97 (dd, 1H, J = 11, 2.7 Hz), 4.89 (t, 1H, J = 10 Hz), 4.83 (d, 1H, J = 9.8 Hz), 4.79-4.72 (m, 1H), 4.68 (br s, 1H), 3.91 (s, 3H), 3.66 (dd, 1H, J = 18, 5.3 Hz), 3.42-3.35 (m, 2H), 3.21 (dd, 1H, J = 13, 4.0 Hz), 3.17 (dd, 1H, J = 15, 5.1 Hz), 3.08 (dd, 1H, J = 15, 7.6 Hz), 2.66-2.57 (m, 2H), 2.47-2.38 (m, 1H), 1.95 (ddd, 1H, J = 14, 12, 4.7 Hz), 1.85-1.77 (m, 1H), 1.75-1.67 (m, 1H), 1.43 (s, 9H), 1.27 (s, 3H), 1.20 (s, 3H), 1.08 (d, 3H, J = 7.0 Hz), 1.03 (d, 3H, J = 6.7 Hz), 0.98 (d, 3H, J = 6.5 Hz).

### (b) Preparation of Cryptophycin 55 glycinate hydrochloride salt

To a solution of the product of step (a) (122 mg, 0.141 mmol) in 471 ml of methylene chloride at room temperature was added a 4.0 M solution of hydrogen chloride in 1,4-dioxane (178 ml, 0.707 mmol). After stirring for lh 20 min, the clear, colorless reaction mixture was concentrated *in vacuo* to provide 120 mg (99%, corrected for 7 wt% dioxane) of the title compound as a white foam: 500 MHz ¹H NMR (MeOH-d₄) d 7.81 (dd, 1H, J = 8.5, 2.2 Hz), 7.46-7.41 (m, 2H), 7.40-7.36 (m, 3H), 7.31 (d, 1H, J = 2.1 Hz), 7.20 (dd, 1H, J = 8.4, 2.1 Hz), 7.01 (d, 1H, J = 8.4 Hz), 6.70 (ddd, 1H, J = 15, 13, 3.7 Hz), 5.97 (dd, 1H, J = 15, 1.7 Hz), 5.55 (d, 1H, J = 9.9 Hz), 5.18 (d, 1H, J = 9.9 Hz), 5.14 (dd, 1H, J = 10, 2.8 Hz), 4.84 (t, 1H, J = 10 Hz), 4.52 (dd, 1H, J = 11, 3.7 Hz), 3.87 (s, 3H), 3.78 (d, 1H, J = 18 Hz), 3.50 (dd, 1H, J = 13, 9.8 Hz), 3.23 (d, 1H, J = 18 Hz), 3.20 (dd, 1H, J = 14, 3.6 Hz), 3.13 (dd, 1H, J = 13, 2.4 Hz), 2.80-2.69 (m, 3H), 2.41-2.32 (m, 1H), 1.99-1.92 (m, 1H), 1.91-1.81 (m, 2H), 1.25 (s, 3H), 1.20 (s, 3H), 1.12 (d, 3H, J = 7.0 Hz), 1.06 (d, 3H, J = 6.2 Hz), 1.04 (d, 3H, 6.2 Hz).

### Example 4

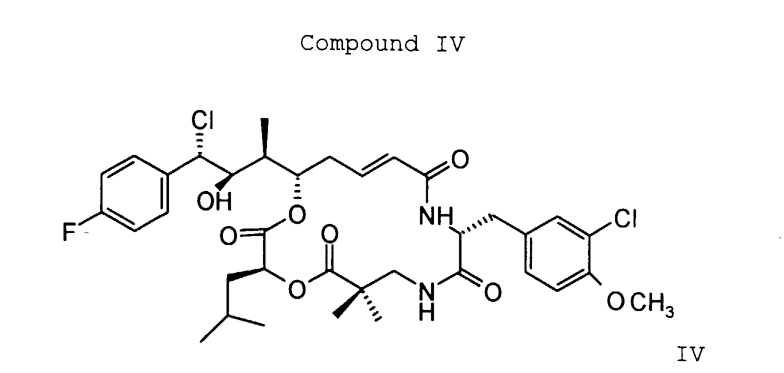

### (a) Preparation of Compound IVA

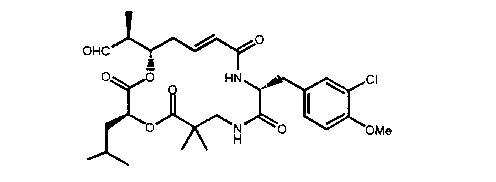

To a solution of Cryptophycin 53 (2.0 g, 2.99 mmol, Barrow, R.A. et al., *J. Am. Chem. Soc*. **1995**, 117, 2479-2490) in 30 mL of DME, was added a 2 M aqueous perchloric acid solution (15 mL, 30 mmol) and the resulting mixture was stirred for 6 hours. Upon careful neutralization with saturated NaHCO₃ (50 mL) the mixture was extracted with CH₂Cl₂ (4 x 100 mL) and the combined organic layers were dried over Na₂SO₄, filtered and concentrated in vacuo. Purification by column chromatography (silica gel, 5% MeOH/CH₂Cl₂) gave a mixture of diols (1.5 g) in 72% yield as a 3:1 anti/syn mixture.

To a solution of the diols (1.0 g, 1.46 mmol), in 20 mL of THF and 15 mL of water, was added NaIO₄ (1.9 g, 8.9 mmol) and the mixture was stirred under nitrogen overnight. Upon removing the bulk of the THF under reduced pressure, the residue was diluted with water (100 mL) and extracted with CH₂Cl₂ (4 x 50 mL). The combined organic extracts were washed with brine (1 x 25 mL), dried over Na₂SO₄, filtered and concentrated under vacuum. Residual benzaldehyde was removed by dissolving the solid in 100 mL of toluene and subsequently removing the toluene at 40 °C on a rotary evaporator. Two additional evaporations from toluene gave the aldehyde as a yellow foam (0.828 g) in 98% yield. The resulting aldehyde (Compound VA) was used without further purification and was stored at -23 °C for stability reasons: [α]²⁰_{D}+23.0 ° (c 0.565, CHCl₃); ¹H NMR (300 MHz, CDCl₃) δ 9.64-9.63 (d, 1 H, *J* = 1.4 Hz), 7.28-7.26 (m, 1 H), 7.21-7.20 (d, 1 H, *J* = 1.9 Hz), 7.08-7.05 (dd, 1 H, *J* = 7.1, 1.7 Hz), 6.87-6.84 (d, 1 H, *J* = 8.5 Hz), 6.82-6.72 (m, 1 H), 5.80-5.75 (d, 1 H, *J* = 15.0 Hz), 5.54-5.51 (d, 1 H, *J* = 7.7 Hz), 5.40-5.33 (m, 1 H), 4.85-4.81 (dd, 1 H, *J* = 9.7, 3.2 Hz), 4.78-4.71 (m, 1 H), 3.88 (s, 3 H), 3.46-3.39 (dd, 1 H, *J* = 13.5, 8.6 Hz), 3.15-3.03 (m, 3 H), 2.68-2.35 (m, 3 H), 1.82-1.63 (m, 2 H), 1.45-1.37 (m, 1 H), 1.24 (s, 3 H), 1.19-1.16 (d, 3 H, *J* = 7.1 Hz), 1.18 (s, 3 H), 0.94-0.92 (d, 3 H, *J* = 6.5 Hz), 0.89-0.87(d, 3 H, *J* = 6.5 Hz); ¹³C NMR (63 MHz, CDCl₃) δ 200.7, 177.8, 170.6, 170.1, 165.1, 153.9, 141.1, 130.7, 129.8, 128.1, 124.9, 122.3, 112.3, 73.4, 71.1, 56.0, 54.6, 49.9, 46.4, 42.7, 39.2, 36.1, 35.2, 24.7, 22.8, 22.7, 21.3, 10.7; IR (CHCl₃) 3422, 2964, 2936, 1755, 1730, 1718, 1678, 1529, 1504, 1487,1474, 1464, 1442, 1320, 1303, 1281, 1259, 1244, 1185, 1151, 1127, 1067 cm⁻¹; Anal. (C₂₉H₃₉ClN₂O₆): C, H, N.

### (b) Preparation of Compound IVB

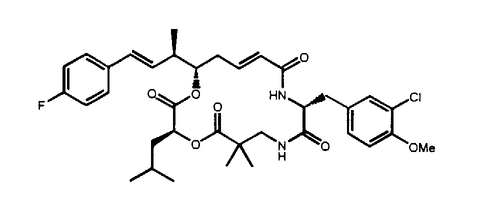

To a slurry of 4-fluorobenzyl triphenylphosphonium bromide (1.4 g, 3.1 mmol) in 15 mL of dry THF at -70 °C under N2 was added a solution of 1.6 M n-butyl lithium in hexanes (1.95 mL, 3.12 mmol) dropwise over 5 minutes. After stirring for 10 minutes, the mixture was allowed to warm to 0 °C, at which point it became homogeneous and deep red-brown in color. This red solution was added dropwise via a double-tipped needle to aldehyde of Example 5, step (a) (1.5 g, 2.6 mmol, Compound IVA) in 15 mL of THF at -70 °C. The reaction mixture was allowed to stir at -70 °C for 30 minutes, then warmed to room temperature over 45 minutes. Saturated NH₄Cl (10 mL) was added followed by EtOAc (150 mL). The EtOAc solution was washed with water (2 x 25 mL) and brine (1 x 20 mL), dried over MgSO₄, filtered through a pad of silica gel and evaporated to a yellow foam. Chromatography (30-100% EtOAc/ hexanes) provided the styrene (1.24 g) in 71% yield as a white foam and as an E/Z mixture.

The mixture of isomers was dissolved in benzene (40 mL) and heated to reflux in the presence of 1,1'-azobis(cyclohexanecarbonitrile) (VAZO) (0.05 g, 0.20 mmol) and thiophenol (0.076 mL, 0.74 mmol) for 24 hours. After concentration the residue was purified by radial PLC (20-80% EtOAc/hexanes) to give the E isomer (1.06 g) as a white foam containing triphenylphosphine oxide by NMR. A 0.15 g sample was purified by reverse-phase HPLC (60:40) CH₃CN:H₂O to give 0.092 g of a pure analytical sample: [α]²⁰_{D} + 27.49 ° (c 1.05, CHCl₃ ); ¹H NMR (300 MHz, CDCl₃) δ 7.31-6.96 (m, 7 H), 6.85-6.83 (d, 1 H, *J* = 8.5 Hz), 6.81-6.74 (m, 1 H), 6.39-6.34 (d, 1 H, *J* = 15.8 Hz), 5.96-5.88 (dd, 1 H, *J* = 8.8, 15.8 Hz), 5.77-5.72 (d, 1 H, *J* = 15.2 Hz), 5.49-5.47 (d, 1 H, *J* = 7.7 Hz), 5.07-5.02 (m, 1 H), 4.86-4.83 (dd, 1 H, *J* = 9.2, 2.5 Hz), 4.82-4.73 (m, 1 H), 3.87 (s, 3 H), 3.45-3.38 (dd, 1 H, *J* = 13.4, 8.5 Hz), 3.14-3.08 (m, 3 H), 2.57-2.52 (m, 2 H), 2.43-2.34 (m, 1 H), 1.71-1.58 (m, 2 H), 1.36-1.29 (m, 1 H), 1.22 (s, 3 H), 1.16 (s, 3 H), 1.14-1.11 (d, 3 H, *J* = 6.9 Hz), 0.78-0.73 (m, 6 H); ¹³C NMR (63 MHz, CDCl₃) δ 177.8, 170.5, 170.3, 165.1, 160.2, 154.0, 142.0, 137.6, 132.9, 132.8, 130.8, 130.4, 130.0, 129.6, 128.2, 127.6, 127.5, 124.6, 122.4, 115.6, 115.2, 112.2, 71.3, 56.0, 54.4, 46.4, 42.7, 42.1, 39.5, 36.4, 35.2, 24.5, 22.8, 22.6, 21.2, 17.2; IR (KBr) 3421, 3289, 2862, 2933, 1751, 1722, 1678, 1604, 1534, 1509, 1259, 1228, 1149, 1066, 1024, 1011, 971, 815 cm⁻¹; Anal. (C₃₆H₄₄ClFN₂O₇) C, H, N.

### (c) Preparation of Compound IVC

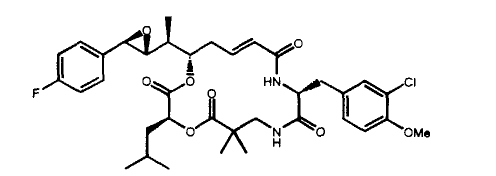

To a solution of the above styrene (0.906 g, 1.35 mmol) in 4.5 mL CH₂Cl₂ at 0 °C was added 3-chloroperoxybenzoic acid (0.25 g, 1.45 mmol) and toluene (2.2 mL) and stirring continued at 0 °C for 30 minutes. The ice-bath was removed and the reaction allowed to stir at room temperature for 23 hours. After diluting with 20 mL of CH₂Cl₂ , the reaction mixture was washed with 10% Na₂S₂O₅ (1 x 10mL), water (1 x 10mL), saturated NaHCO₃ (1 x 10mL) and brine (1 x 10mL) and finally was dried over Na₂SO₄. Filtration and concentration gave 0.814 g of the product as a mixture of the b/a epoxides. A 0.23 g portion was purified by reverse-phase HPLC (CH₃CN/H₂O) to give 0.073 g of the β-epoxide (Compound IVC) as a white foam: [α]²⁰_{D} + 25.6 ° (c 0.626, CHCl₃ ); ¹H NMR (300 MHz, CDCl₃) δ 7.26-7.03 (m, 7 H), 6.85-6.83 (d, 1 H, *J* = 8.4 Hz), 6.82-6.72 (m, 1 H), 5.74-5.69 (d, 1 H, *J* = 15.2 Hz), 5.44-5.42 (d, 1 H, *J* = 7.9 Hz), 5.23-5.18 (m, 1 H), 4.85-4.81 (dd, 1 H, *J* = 9.7, 2.9 Hz), 4.77-4.73 (m, 1 H), 3.88 (s, 3 H), 3.66 (s, 1 H), 3.46-3.39 (dd, 1 H, *J* = 13.5, 8.8 Hz), 3.12-3.07 (m, 3 H), 2.89-2.87 (dd, 1 H, *J* = 1.5, 7.7 Hz), 2.60-2.54 (m, 1 H), 2.49-2.41 (m, 1 H), 1.81-1.65 (m, 3 H), 1.34-1.25 (m, 1 H), 1.22 (s, 3 H), 1.16 (s, 3 H), 1.15-1.13 (d, 3 H, *J* = 7.0 Hz), 0.87-0.82 (m, 6 H); ¹³C NMR (63 MHz, CDCl₃) δ 177.8, 170.3, 164.9, 164.7, 154.0, 141.6, 137.8, 132.4, 130.7, 129.6, 128.1, 127.3, 127.2, 124.6, 122.4, 115.8, 115.5, 112.2, 75.8, 71.0, 63.0, 58.2, 56.0, 54.4, 46.3, 42.7, 40.4, 39.3, 36.7, 35.2, 23.5, 22.8, 22.76, 22.6, 21.1, 13.3; IR (CHCl₃) 3426, 3030, 3006, 2964, 2936, 1752, 1711, 1683, 1608, 1514, 1485, 1442, 1303, 1281, 1259, 1188, 1155, 1067, 838 cm⁻¹; Anal. (C₃₆H₄₄ClFN₂O₈) C, H, N.

### (d) Preparation of Compound IV

A 4 M solution of HCl in dioxane (0.4 mL, 1.6 mmol) was added dropwise over 5 minutes to a -70 °C solution of β-epoxide (Compound VC) (0.44 g, 0.64 mmol) in 30 mL CH₂Cl₂. Following 2 additional hrs of stirring at -70 °C, the solution was concentrated in vacuo. The crude product was purified by radial PLC (silica gel, 30-50-100% EtOAc/ CH₂Cl₂) followed by reverse phase HPLC (50:50) CH₃CN:H₂O to give 0.152 g (33%) of the desired chlorohydrin (Compound V) as a white foam: [α]²⁰_{D} + 60.0 ° (c 2.62, CHCl₃ ); ¹H NMR (300 MHz, CDCl₃) δ 7.41-7.05 (m, 7 H), 6.87-6.84 (d, 1 H, J = 8.4 Hz), 6.83-6.77 (m, 1 H), 5.80-5.75 (d, 1 H, *J* = 15.4 Hz), 5.52-5.49 (d, 1 H, *J* = 7.8 Hz), 5.13-5.21 (m, 1H), 4.94-4.90 (dd, 1H, *J* = 9.7,3.2 Hz), 4.75-4.72 (m, 1 H), 4.67-4.63 (d, 1 H, *J* = 9.5 Hz), 4.00-3.95 (m, 1H), 3.89 (s, 3 H), 3.42-3.35 (dd, 1H, *J* = 8.3, 13.5 Hz), 3.20-3.02 (m, 3 H), 2.71-2.65 (m, 1 H), 2.49-2.37 (m, 2 H), 1.82-1.63 (m, 2 H), 1.51-1.38 (m, 2 H), 1.23 (s, 3 H), 1.17 (s, 3 H), 1.04-1.02 (d, 3 H, *J* = 7.0 Hz). 0.97-0.85 (m, 6 H); ¹³C NMR (63 MHz, CDCl₃) δ 177.6, 170.5, 170.3, 165.3, 160.7, 153.9, 142.2, 137.5, 134.5, 130.8, 129.8, 129.7, 128.2, 124.6, 122.2, 76.1, 74.0, 71.1, 61.4, 56.1, 54.5, 46.4, 42.7, 39.6, 38.4, 36.3, 35.1, 24.8, 23.0, 22.9, 22.7, 21.5, 8.6; IR (CHCl₃) 3423, 2965, 2935, 2873, 1751, 1715, 1679, 1607, 1528, 1504, 1485, 1464, 1442, 1302, 1281, 1193, 1159, 1152, 1127, 1067 cm⁻¹; Anal. (C₃₆H₄₅Cl₂N₂O₈) C, H, N. IC₅₀ (CEM cell line) 0.033 nM.

### Example 5

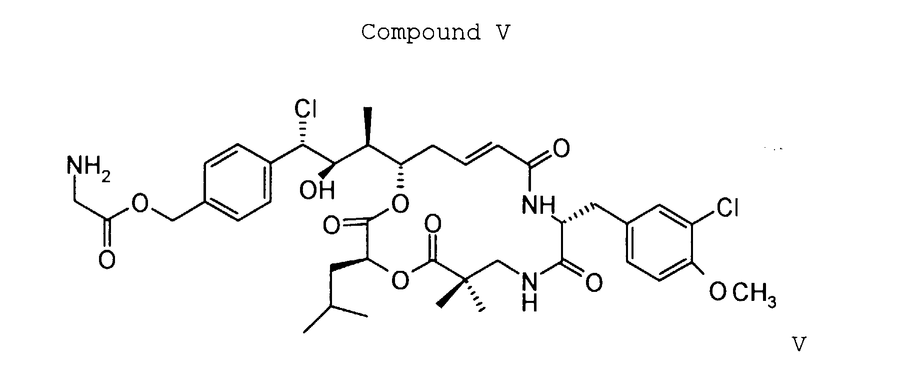

### (a) Preparation of Compound VA

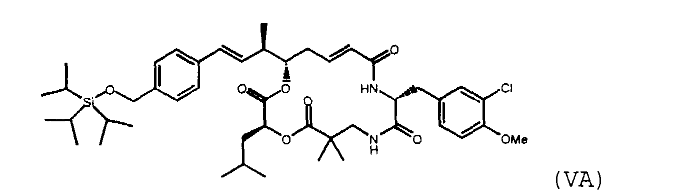

To 4-(triisopropylsiloxymethyl)benzyl triphenylphosphonium bromide (7.6 g, 12.2 mmol) in 100 mL of THF at -50 °C was added dropwise 8.0 mL of a 1.5 M solution of n-butyllithium (8.1 mL, 12.2 mmol). The mixture was warmed slowly to room temperature and stirred for an additional 30 min. To aldehyde of Example 4, step a (Compound IVA) (2.95 g, 5.1 mmol), in 100 mL of THF and at -78 °C, was added dropwise the red ylide solution via a double tipped needle. The resulting mixture was stirred at -78 °C for 3 h and at room temperature for 45 min. Saturated NH₄Cl (100 mL) was added along with ethyl acetate (100 mL), the layers separated and the aqueous one extracted with ethyl acetate (2 x 50 mL). The combined organic layers were washed with water (3 x 40 mL) and brine, dried over MgSO₄, filtered and concentrated in vacuo. The resulting yellow residue was purified using column chromatography (silica gel, 10-20-50% EtOAc/hexanes) to give 3.6 g (84%) of the desired styrene as a white solid and as a mixture of E:Z isomers.

The mixture of isomers (7.3 g, 8.7 mmol) was dissolved in 240 mL of benzene and heated to reflux in the presence of 1,1'-azobis(cyclohexanecarbonitrile) (VAZO) (0.32 g, 0.87 mmol) and thiophenol (3.7 mL, 4.0 mmol). Following 5 h of reflux, the solution was concentrated and the residue purified by column chromatography (silica gel, 5-50% EtOAc/hexanes) to give 6.7 g (92%) of the E isomer as a white solid: [α]²⁰_{D} +31.9 ° (c1.0, CHCl₃); ¹H NMR (300 MHz, CDCl₃) δ 7.3-7.22 (m, 5 H), 7.20-7.19 (d, 1 H, *J* = 1.95 Hz), 7.07-7.04 (dd, 1 H, *J* = 8.4, 2.0 Hz), 6.85-6.82 (d, 1 H, *J* = 8.5 Hz), 6.8-6.7 (m, 1 H), 6.4-6.38 (d, 1 H, *J* = 15.8 Hz), 6.02-5.94 (dd, 1 H, *J* = 15.8, 8.8 Hz), 5.77-5.72 (d, 1 H, *J* = 14.9 Hz), 5.56-5.54 (d, 1 H, *J* = 7.9 Hz), 5.1-4.7 (m, 5 H), 3.9 (s, 3 H), 3.45-3.37 (dd, 1 H, *J* = 13.5, 8.5 Hz), 3.2-3.0 (m, 3 H), 2.6-2.3 (m, 3 H), 1.7-1.5 (m, 2 H), 1.4-1.0 (m, 31 H), 0.75-0.71 (t, 6 H, *J* = 6.1 Hz); ¹³C NMR (63 MHz, CDCl₃) δ 177.8, 170.5, 170.4, 165.2, 153.9, 142.1, 141.1, 135.2, 131.5, 130.8, 129.7, 129.6, 128.1, 125.9, 124.5, 122.4, 112.2, 77.0, 71.4, 64.7, 56.0, 54.4, 46.4, 42.7, 42.2, 39.4, 36.5, 35.3. 24.5, 22.8, 22.6, 22.5, 21.2, 17.9, 17.2, 11.9; IR (CHCl₃) 3423, 2962, 2945, 2867, 1746, 1712, 1681, 1652, 1528, 1503, 1485, 1473, 1464, 1303, 1259 cm⁻¹; Anal. (C₄₆H₆₇ClN₂O₈Si): C, H, N.

### (b) Preparation of Compound VB

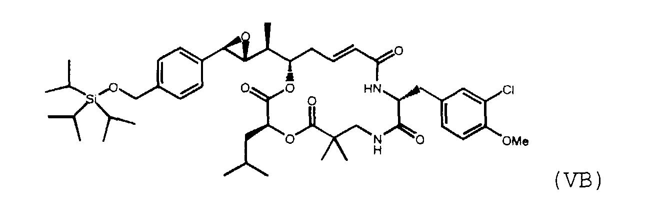

3-Chloroperoxybenzoic acid (0.27 g, 1.59 mmol) was added to a 0 °C solution of styrene of Example 5, step a (Compound VA) (1.25 g , 1.49 mmol) in 20 mL of CH₂Cl₂. The solution was stirred for 1 h at 0 °C and overnight at room temperature. It was concentrated in vacuo and the resulting epoxides separated by reverse phase HPLC to yield 0.67 g of the β epoxide (Compound VIB) (57%) as a white solid: [α]²⁰_{D} +20.9 ° (c 0.765, CHCl₃); ¹H NMR (300 MHz, CDCl₃) 6 7.35-7.33 (d, 2 H, *J* = 7.8 Hz), 7.26-7.2 (m, 4 H), 7.05-7.02 (bd, 1 H, *J* = 8.2 Hz), 6.84-6.81 (d, 1 H, *J* = 8.4 Hz), 6.81-6.65 (m, 1 H), 5.8-5.65 (m, 2 H), 5.25-5.15 (m, 1 H), 4.9-4.7 (m, 4 H), 3.9 (s, 3 H), 3.7 (s, 1 H), 3.46-3.42 (dd, 1 H, *J* = 13.4, 8.8 Hz), 3.15-3.0 (m, 3 H), 2.93-2.9 (d, 1 H, *J* = 7.3 Hz), 2.6-2.4 (m, 2 H), 1.8-1.6 (m, 3 H), 1.4-1.0 (m, 31 H), 0.83-0.79 (t, 6 H, *J* = 5.3 Hz); ¹³C NMR (63 MHz, CDCl₃) δ 177.7, 170.5, 170.4, 165.1, 153.9, 142.1, 141.6, 136.7, 135.1, 130.7, 129.8, 128.1, 125.9, 125.5, 124.6, 122.3, 112.2, 75.9, 71.0, 64.6, 63.0, 58.9, 56.0, 54.6, 46.3, 42.7, 40.5, 39.2, 36.8, 35.2. 24.2, 22.8, 22.7, 22.6, 18.0, 13.4, 11.9; IR (CHCl₃) 3424, 2962, 2945, 2867, 1751, 1712, 1682, 1528, 1503, 1485, 1473, 1464, cm⁻¹; Anal. (C₄₆H₆₇ClN₂O₉Si): C, H, N. IC₅₀ (CEM cell line) 0.7 nM.

### (c) Preparation of Compound VC

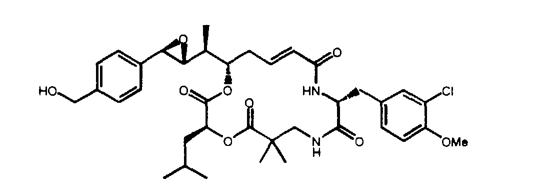

Tetrabutylammonium fluoride (0.14 mL, 0.14 mmol), as a 1.0 M solution in THF, was added dropwise to a 0 °C solution of the β epoxide of Example 5, step b (Compound VIB) ( 0.1 g, 0.117 mmol) in 3.5 mL of THF. The solution was allowed to-warm up to room temperature and stirring was continued for another 20 min, followed by the addition of water (10 mL) and ethyl acetate (20 mL). The layers were separated and the aqueous one was extracted with CH₂Cl₂ (3 x 20 mL). The combined organic layers were dried over anhydrous Na₂SO₄, filtered and concentrated in vacuo to yield the free alcohol. Purification by column chromatography (silica gel, 70-100% EtOAc-hexanes) yielded 0.068 g (84%) of the pure alcohol as a white solid: [α]²⁰_{D} +26.2 ° (c0.435, CHCl₃); ¹H NMR (300 MHz, CDCl₃) δ 7.39-7.36 (d, 2 H, *J* = 7.8 Hz), 7.26-7.23 (d, 3 H, *J* = 9.1 Hz), 7.18 (s, 1 H), 7.05-7.02 (d, 1 H, *J* = 8.5 Hz), 6.85-6.82 (d, 1 H, *J* = 8.2 Hz), 6.82-6.7 (m, 1 H), 5.72-5.67 (d, 1 H, *J* = 15.1 Hz), 5.55-5.52 (d, 1 H, *J* = 7.8 Hz), 5.22-5.17 (m, 1 H), 4.85-4.7 (m, 4 H), 3.9 (s, 3 H), 3.7 (s, 1 H), 3.45-3.38 (dd, 1 H, *J* = 13.4, 9.3 Hz), 3.2-3.0 (m, 3 H), 2.92-2.89 (d, 1 H, *J* = 7.6 Hz), 2.65-2.4 (m, 2 H), 1.8-1.6 (m, 4 H), 1.4-1.2 (m, 1 H), 1.22 (s, 3 H), 1.16 (s, 3 H), 1.16-1.13 (d, 3 H, *J* = 7.2 Hz), 0.86-0.82 (t, 6 H, *J* = 6.5 Hz); ¹³C NMR (63 MHz, CDCl₃) δ 177.8, 171.0, 170.4, 165.5, 153.8, 141.5, 141.4, 135.7, 133.5, 130.6, 130.0, 128.0, 127.1, 125.6, 124.6, 122.2, 112.3, 77.2, 76.5, 76.0, 71.0, 64.2, 63.1, 58.8, 56.0, 54.7, 46.3, 42.7, 40.5, 39.3, 36.9, 35.1, 24.5, 22.7, 22.5, 22.1, 13.4; IR (CHCl₃) 3422, 2992, 2963, 2936, 2874, 1751, 1713, 1682, 1651, 1504, 1486, 1303, 1259, 1186, 1165, 1151, 1067 cm⁻¹; FAB HRMS [M + H] cacld for (C₃₇H₄₈ClN₂O₉) 699.3048, found 699.3054. IC50 (CEM cell line) 0.004 nM.

### (d) Preparation of Compound VD

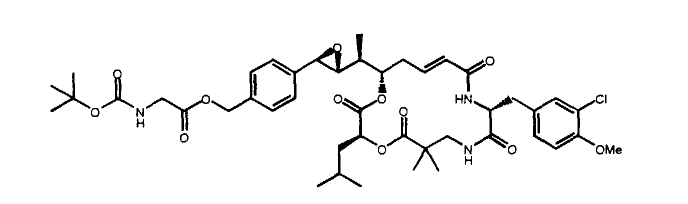

To a 0 °C solution of alcohol of Example 5, step c (Compound VC) (0.08 g, 0.114 mmol), *N*-(tert-butoxycarbonyl)glycine (0.034 g, 0.194 mmol) and 4-dimethylaminopyridine (DMAP) (0.004 g, 0.034 mmol) in 2.0 mL CH₂Cl₂ was added 1,3-dicyclohexylcarbodiimide (DCC) (0.040 g, 0.194 mmol). The mixture was stirred at 0 °C for 10 min and at room temperature for 45 min, filtered and concentrated in vacuo. The resulting residue was purified using column chromatography (silica gel, 70-80% EtOAc/hexanes) to give 0.07 g (72%) of the ester as a white solid: [α]²⁰_{D}+18.5 ° (c 0.65, CHCl₃); ¹H NMR (300 MHz, CDCl₃) δ 7.4-7.2 (m, 6 H), 7.11-7.08 (dd, 1 H, *J* = 8.4, 1.8 Hz), 6.9-6.87 (d, 1 H, *J* = 8.4 Hz), 6.86-6.7 (m, 1 H), 5.78-5.73 (d, 1 H, *J* = 15.2 Hz), 5.64-5.62 (d, 1 H, *J* = 7.4 Hz), 5.3-5.22 (m, 1 H), 5.22 (s, 2 H), 5.1-5.0 (bs, 1 H), 4.9-4.7 (m, 2 H), 4.0-3.99 (d, 2 H, *J* = 5.4 Hz), 3.9 (s, 3 H), 3.73-3.72 (d, 1 H, *J =* 1.0 Hz), 3.5-3.43 (dd, 1 H, *J* = 13.4, 8.6 Hz), 3.2-3.0 (m, 3 H), 2.95-2.92 (d, 1 H, *J* = 6.4 Hz), 2.65-2.4 (m, 2 H), 1.8-1.6 (m, 3 H), 1.5 (s, 9 H), 1.45-1.3 (m, 1 H), 1.26 (s, 3 H), 1.2 (s, 3 H), 1.2-1.17 (d, 3 H, *J* = 8.7 Hz), 0.9-0.86 (t, 6 H, *J* = 6.3 Hz); ¹³C NMR (63 MHz, CDCl₃) δ 177.7, 170.6, 170.3, 170.2, 165.1, 155.6, 153.8, 141.4, 137.1, 135.6, 130.6, 129.9, 128.6, 128.0, 125.7, 124.7, 122.2, 112.2, 79.9, 75.8, 70.9, 66.4, 63.1, 58.5, 56.0, 54.7, 48.9, 46.3, 42.7, 42.4, 40.5, 39.3, 36.8, 35.2, 28.2, 24.5, 22.8, 22.7, 22.6, 21.2, 13.5; Anal. (C₄₄H₅₈ClN₃O₁₂): C, H, N. IC₅₀ (CEM cell line) 0.0055 nM.

### (e) Preparation of Compound VE

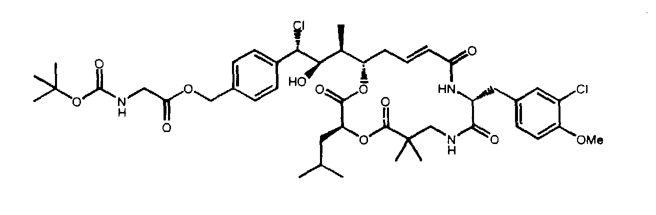

Trimethylsilyl chloride (0.09 mL, 0.75 mmol) was added to a -60 °C solution of the above β-epoxide (0.16 g, 0.187 mmol) in 5.0 mL of CHCl₃. Following 2 h of stirring between -60 °C to -40 °C an additional 0.09 mL of TMSCl was added and stirring continued for 3 h. The solution was allowed to warm up to room temperature, concentrated and purified by reverse phase preparative HPLC (55:45) CH₃CN:H₂O to separate the two resulting chlorohydrins. This purification gave 0.058 g (35%) of the desired chlorohydrin: [α]²⁰_{D} + 50.5 ° (*c* 1.075, CHCl₃ ); ¹H NMR (300 MHz, CDCl₃) δ 7.42-7.2 (m, 6 H), 7.13-7.09 (dd, 1 H, *J* = 8.4, 1.8 Hz), 6.9-6.87 (d, 1 H, *J* = 8.4 Hz), 6.85-6.7 (m, 1 H), 5.9-5.8 (m, 2 H), 5.2 (s, 3 H), 5.15-5.05 (m, 1 H), 5.0-4.9 (m, 1 H), 4.8-4.72 (m, 1 H), 4.71-4.68 (d, 1 H, *J* = 9.7 Hz), 4.07-4.03 (d, 1 H, *J* = 9.3 Hz), 3.99-3.97 (d, 2 H, *J* = 5.5 Hz), 3.9 (s, 3 H), 3.44-3.37 (dd, 1 H, *J =* 13.6, 8.3 Hz), 3.23-3.14 (m, 2 H), 3.08-3.0 (dd, 1 H, *J =* 14.5, 8.0 Hz), 2.75-2.4 (m, 3 H), 2.0-1.7 (m, 3 H), 1.5 (s, 10 H), 1.26 (s, 3 H), 1.21 (s, 3 H), 1.08-1.06 (d, 3 H, *J* = 7.0 Hz), 0.977-0.963 (d, 3 H, *J* = 4.0 Hz), 0.956-0.942 (d, 3 H, *J* = 4.1 Hz); ¹³C NMR (63 MHz, CDCl₃) 177.5, 170.5, 170.2, 170.1, 165.3, 153.9, 142.2, 139.0, 138.3, 136.1, 130.8, 129.9, 128.7, 128.2, 128.1, 124.5, 122.3, 112.2, 80.0, 76.1, 73.9, 71.1, 66.2, 61.7, 56.1, 54.6, 46.4, 42.7, 42.3, 39.6, 38.4, 36.3, 35.1, 28.2, 24.8, 23.0, 22.9, 22.7, 21.5, 8.6; IR (CHCl₃) 3428, 3009, 2966, 2935, 1750, 1714, 1683, 1504, 1486, 1369, 1259, 1193, 1162, 1127, 1067; FAB HRMS [M + H] cacld for (C₄₄H₆₀ClN₃O₁₂) 892.3554, found 892.3565. IC₅₀ (CEM cell line) 0.013 nM.

### (f) Preparation of Compound V

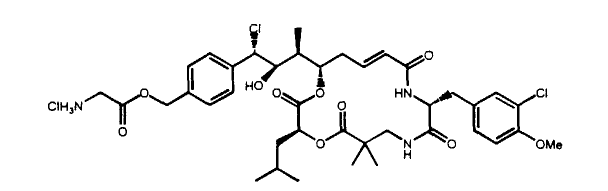

A 4 M solution of hydrogen chloride in 1,4-dioxane (0.08 mL, 0.33 mmol) was added to a solution of the glycinate (0.058 g, 0.065 mmol) in 0.2 mL of CH₂Cl₂. The resulting mixture was stirred at room temperature for 3 h, concentrated in vacuo and maintained under vacuum for 3 days to remove the 1,4-dioxane thus giving the desired hydrochloride salt in quantitative yield: [α]²⁰_{D} + 26.2 ° (c 0.58, MeOH ); ¹H NMR (500 MHz, CD₃OD) 6 7.48-7.42 (q, 4 H, *J =* 11.2 Hz), 7.31-7.3 (d, 1 H, *J* = 2.0 Hz), 7.21-7.19 (dd, 1 H, *J* = 8.5, 2.0 Hz), 7.01-7.0 (d, 1 H, *J* = 8.4 Hz), 6.8-6.7 (m, 1 H), 6.0-5.95 (dd, 1 H, *J =* 15.2, 1.5 Hz), 5.3 (d, 2 H, *J* = 1.3 Hz), 5.16-5.1 (m, 1 H), 5.09-5.07 (dd, 1 H, *J =* 10.0, 3.6 Hz), 4.84-4.82 (d, 1 H, *J* = 9.8 Hz), 4.54-4.51 (dd, 1 H, *J =* 11.3, 3.7 Hz), 4.05-4.03 (dd, 1 H, *J* = 9.5, 1.8 Hz), 3.9 (s, 2 H), 3.86 (s, 3 H), 3.5-3.47 (d, 1 H, *J* = 13.5 Hz), 3.22-3.18 (dd, 1 H, *J* = 14.5, 3.6 Hz), 3.14-3.11 (d, 1 H, *J* = 13.5 Hz), 2.8-2.77 (d, 1 H, *J* = 14.4 Hz), 2.78-2.75 (m, 2 H), 2.55-2.35 (m, 2 H), 1.9-1.55 (m, 4 H), 1.4-1.3 (m, 1 H), 1.24 (s, 3 H), 1.2 (s, 3 H), 1.04-1.03 (d, 3 H, *J* = 7.0 Hz), 1.02-1.0 (t, 6 H, *J* = 7.2 Hz); ¹³C NMR (75 MHz, CDCl₃) δ 178.9, 173.8, 171.9, 168.3, 155.3, 144.2, 141.8, 136.7, 132.3, 131.5, 129.75, 129.7, 129.4, 125.2, 123.3, 113.5, 77.2, 74.7, 72.6, 68.5, 63.5, 57.6, 56.7, 47.6, 44.1, 41.1, 40.4, 37.9, 36.5, 26.3, 23.6, 23.5, 22.2, 9.0; IR (KBr) 3412, 2961, 2935, 1752, 1722, 1669, 1504, 1473, 1279, 1259, 1207, 1151, 1126, 1065 cm⁻¹; FAB HRMS [M - Cl] cacld for (C₃₉H₅₂Cl₂N₃O₁₀) 792.3030, found 792.3020.

## Claims

1. A composition useful for the treatment of a condition selected from the group consisting of cancer, neoplasm, and hyperproliferative cell growth, comprising a compound selected from the group consisting of Compound I, Compound II, Compound III, Compound IV and Compound V 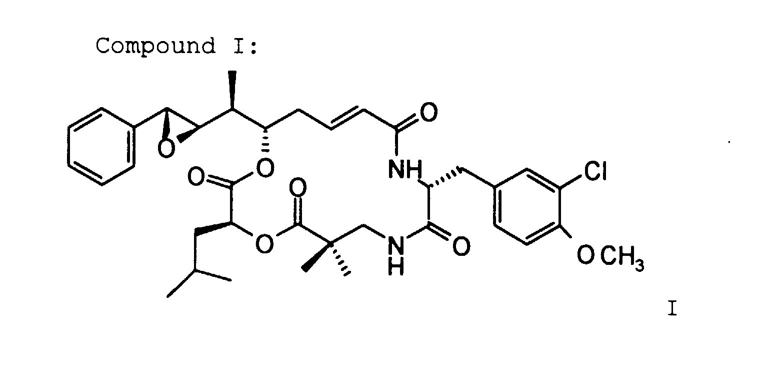 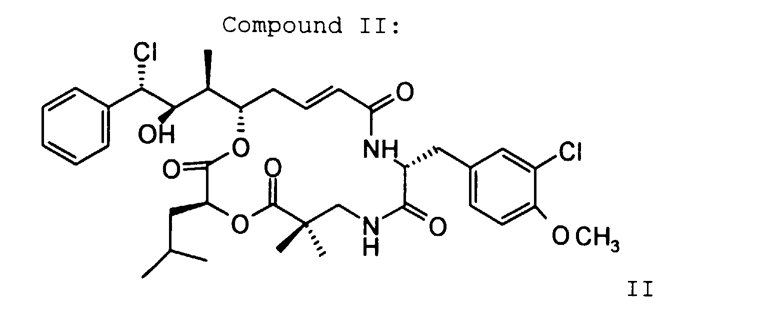 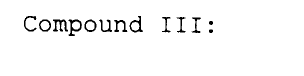 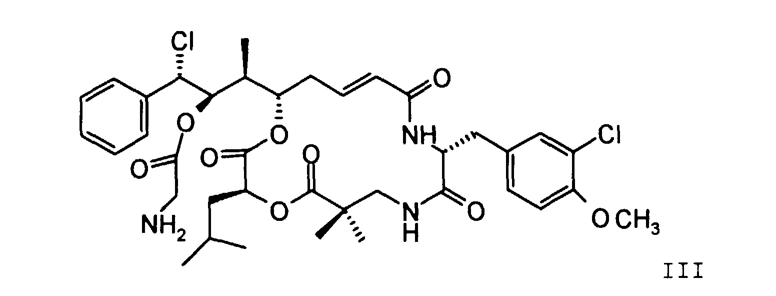 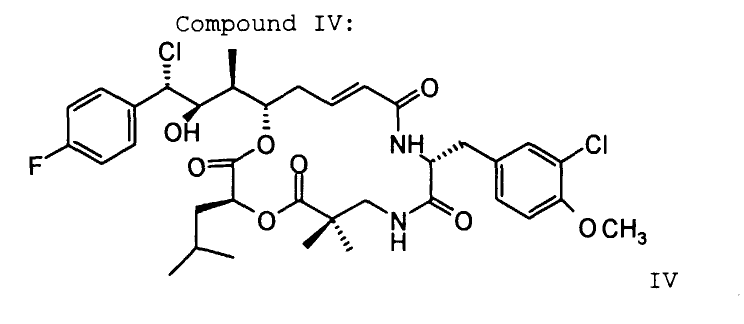 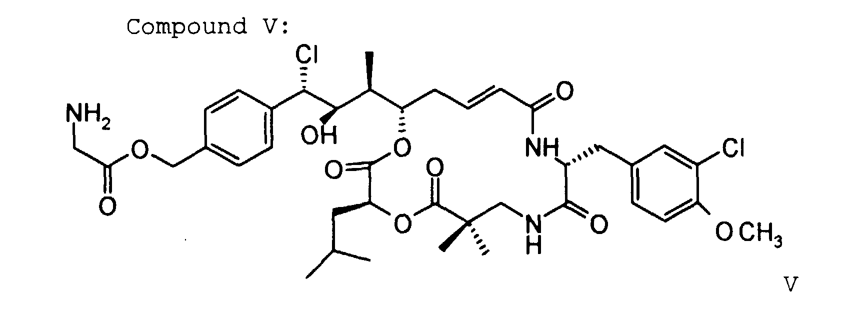 or a pharmaceutically acceptable salt thereof; and one or more a microtubule synergizing agent.

2. A composition of Claim 1 wherein the condition is cancer in a mammal.

3. A composition of Claim 1 wherein the compound is Compound I.

4. A composition of claim 1 wherein the compound is Compound II.

5. A composition of claim 1 wherein the compound is Compound III.

6. A composition of claim 1 wherein the compound is Compound IV.

7. A composition of claim 1 wherein the compound is Compound V.

8. A composition of Claim 1 wherein the microtubule synergizing agent is estramustine.

9. A composition of Claim 8 wherein the compound is Compound I.

10. The use of a compound selected from the group consisting of Compound I, Compound II, Compound III, Compound IV and Compound V 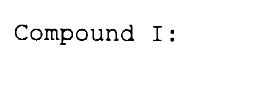 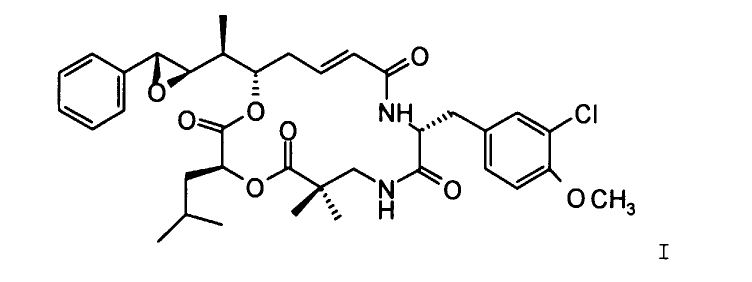 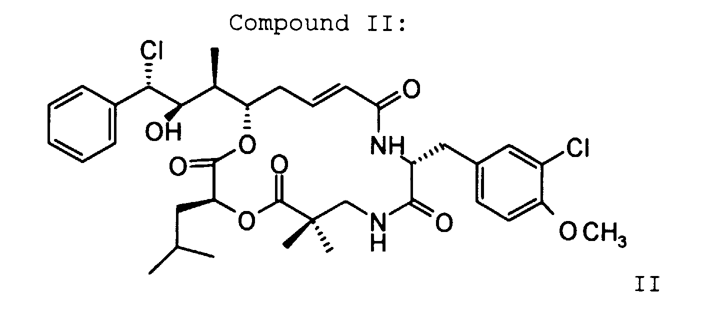 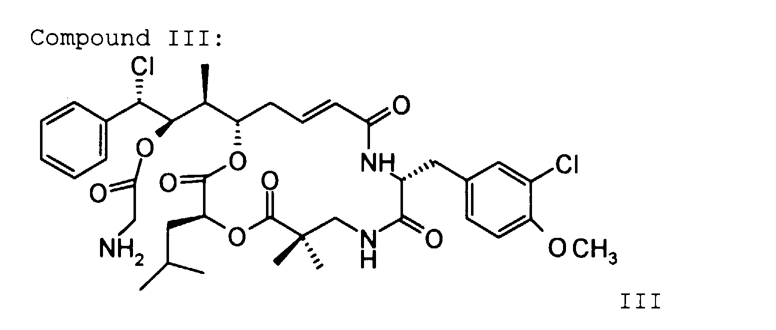 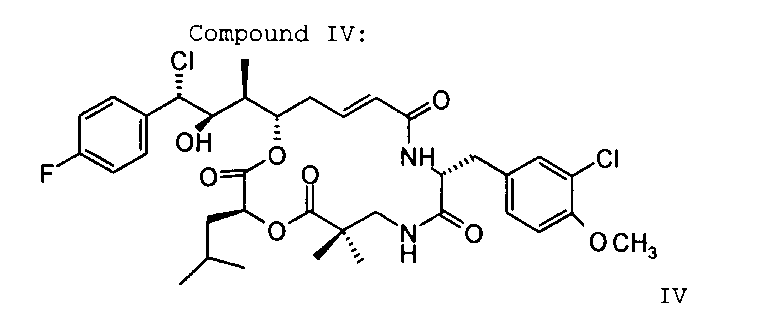 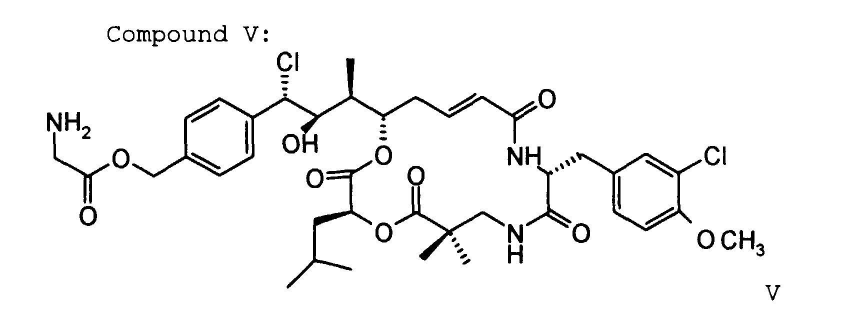 or a pharmaceutically acceptable salt thereof; and one or more a microtubule synergizing agents in the preparation of medicament useful for the treatment of cancer.

11. Use of a compound selected from the group consisting of Compound I, Compound II, Compound III, Compound IV and Compound V 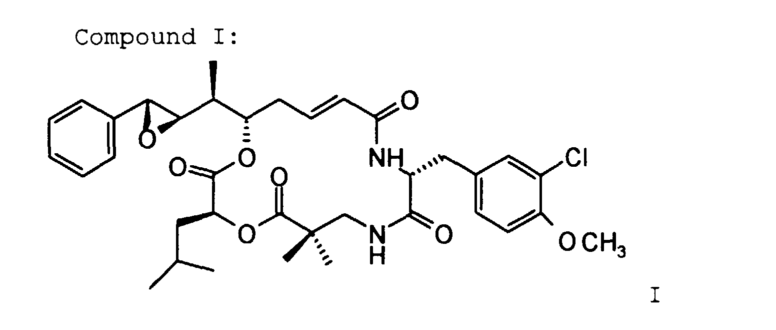 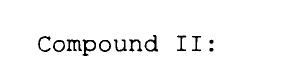 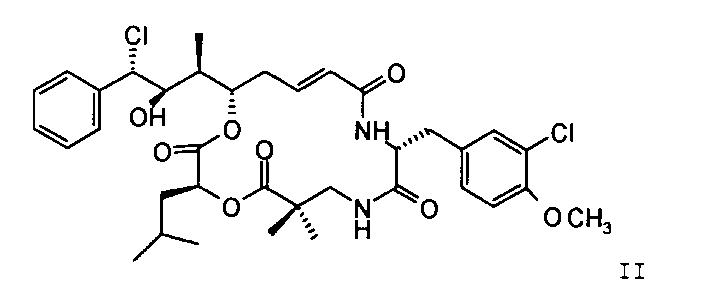 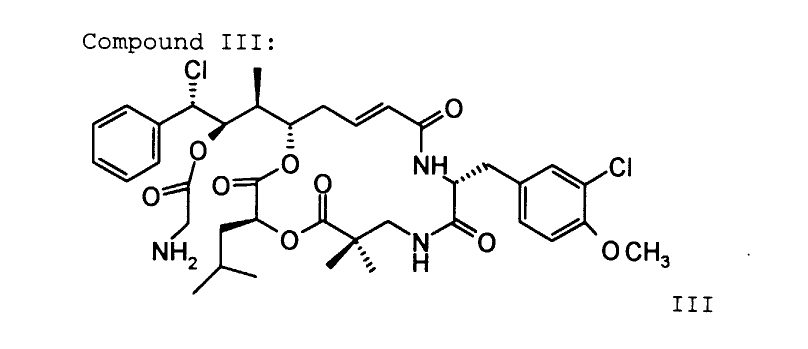 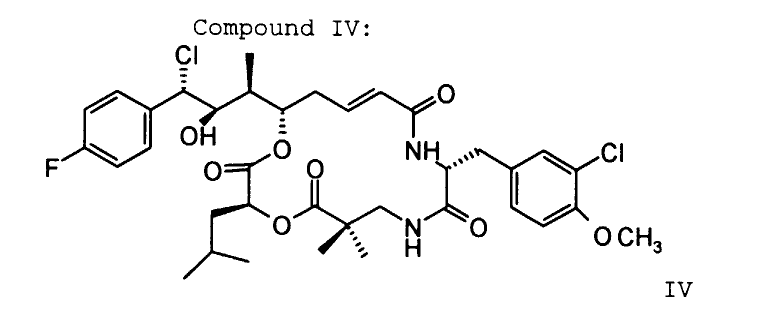 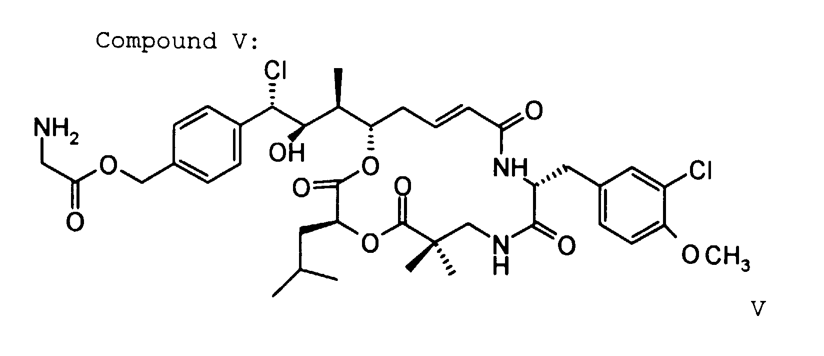 or a pharmaceutically acceptable salt thereof in the manufacture of a medicament for treating cancer for administering prior to or at the time of radiation treatment.

12. Use according to Claim 11 wherein said Compound I, Compound II, Compound III, Compound IV or Compound V is administered within twenty four hours of radiation therapy.

13. Use according to Claim 12 wherein said Compound I, Compound II, Compound III, Compound IV or Compound V is administered within eight hours of radiation therapy.

14. Use according to Claim 13 wherein said Compound I, Compound II, Compound III, Compound IV or Compound V is administered within one hour of radiation therapy.

15. Use of a compound selected from related cryptophycin compounds in the manfacture of a medicament for the treatment of cancer prior to or at the time of radiation treatment.

16. Use according to Claim 15 wherein the related cryptophycin compound is selected from the group consisting of Cryptophycin 1 and Cryptophycin 8.

17. A composition for the treatment of cancer comprising a related cryptophycin compound and one or more a microtubule synergizing agents.

18. A composition of Claim 17 wherein the related cryptophycin compound is selected from Cryptophycin 1 and Cryptophycin 8.
